# FASCICULE DE BREVET EUROPEEN

(11) **EP 3 727 191 B1**
(45) Date de publication et mention de la délivrance du brevet: **12.07.2023**
(21) Numéro de dépôt: 18833971.7
(22) Date de dépôt: 18.12.2018
(51) Int. Cl.: A61D 19/02, A61B 1/303, A61B 1/018, A61B 1/05, A61B 1/06

(54) **APPAREIL D'AIDE À LA PÉNÉTRATION VAGINALE PRÉVU POUR RECEVOIR UN OUTIL DE TRAVAIL**
GERÄT ZUR UNTERSTÜTZUNG DER VAGINALEN PENETRATION UND ZUR AUFNAHME EINES ARBEITSWERKZEUGS
APPLIANCE FOR ASSISTING IN VAGINAL PENETRATION AND PROVIDED TO RECEIVE A WORKING TOOL

(30) Priorité: 18.12.2017 FR 1762339
(43) Date de publication de la demande: 28.10.2020
(73) Titulaire: IMV Technologies, 61300 Saint-Ouen-sur-Iton (FR)
(72) Inventeur: SCHMITT, Eric, 53700 Villaines-la-Juhel (FR); GORGES, Jean-Charles, 72610 Chenay (FR); GUYOMAR, Hélène, 61560 Saint Germain de Martigny (FR)
(74) Mandataire: Santarelli
(86) Numéro de dépôt international: PCT/FR2018/053369
(87) Numéro de publication internationale: WO 2019/122685

(56) Documents cités:
- WO-A1-2010/139912
- WO-A1-2016/066962
- WO-A1-2017/129929
- FR-A1- 2 724 308

## Description

### DOMAINE DE L'INVENTION

L'invention a trait à un appareil d'aide à la pénétration vaginale, en particulier des animaux d'élevage, prévu pour recevoir un outil de travail, en particulier un pistolet d'insémination à utiliser avec une paillette pour la conservation d'une dose prédéterminée de substance à base liquide, notamment de la semence animale pure ou diluée.

### ARRIERE-PLAN TECHNOLOGIQUE

On connait déjà par la demande PCT WO 2016/066962, à laquelle correspond la demande de brevet américain US 2017/0319317, un appareil d'aide à la pénétration vaginale prévu pour recevoir un outil de travail, comportant un manche et un tube speculum s'étendant axialement depuis le manche jusqu'à une extrémité distale où il présente une ouverture. L'appareil comporte en outre un système de vision vidéo comportant un objectif disposé dans le tube spéculum et un dispositif de transmission relié à l'objectif et reliable à un écran déporté de visualisation de l'image obtenue par l'objectif. L'appareil comporte aussi un support d'objectif pour maintenir l'objectif dans une position prédéterminée par rapport au tube speculum dans laquelle l'objectif est au voisinage de l'ouverture du tube speculum et en regard de l'espace situé au-delà de cette ouverture.

Le manche est en outre configuré pour guider à coulissement l'outil de travail dans le tube speculum de sorte que l'outil de travail peut atteindre une position avancée dans laquelle une partie de l'outil de travail saille au-delà de l'ouverture du tube speculum.

Pour pratiquer une insémination artificielle, on choisit un outil de travail comprenant un pistolet d'insémination équipé d'une paillette remplie de semence. Le tube spéculum est introduit dans le vagin de l'animal et une fois que l'appareil est correctement positionné, l'outil est poussé vers la position avancée afin que l'embout de la gaine du pistolet s'introduise au travers du col de l'utérus, puis la semence est éjectée dans l'utérus.

Auparavant, l'appareil (et plus précisément le dispositif de transmission du système de vision vidéo) est relié à un écran déporté tel que l'écran d'un smartphone. Au cours de l'opération, l'opérateur peut observer sur l'écran déporté la progression du tube speculum, puis du pistolet d'insémination, grâce au système de vision vidéo qui renvoie en temps réel l'image de l'espace situé au-delà de l'ouverture du tube speculum.

### OBJET DE L'INVENTION

L'invention vise à améliorer les conditions d'utilisation d'un tel appareil.

L'invention propose à cet effet un appareil d'aide à la pénétration vaginale prévu pour recevoir un outil de travail, comportant :
- un manche ;
- un tube speculum s'étendant axialement depuis le manche jusqu'à une extrémité distale où il présente une ouverture ;
- un système de vision vidéo comportant un objectif disposé dans le tube speculum et un dispositif de transmission relié à l'objectif et reliable à un écran déporté de visualisation de l'image obtenue par ledit objectif ; et
- un support d'objectif pour maintenir l'objectif dans une position prédéterminée par rapport au tube speculum dans laquelle l'objectif est au voisinage de ladite ouverture du tube speculum et en regard de l'espace situé au-delà de ladite ouverture du tube spéculum ;
caractérisé en ce que ledit tube speculum comporte une partie d'extrémité et une partie principale s'étendant entre ladite partie d'extrémité et ledit manche, ladite partie d'extrémité étant disposée autour dudit support d'objectif et délimitant ladite ouverture, ladite partie d'extrémité étant en matière élastiquement déformable et ladite partie principale étant en matière rigide.

Etant donné que la partie d'extrémité en matière élastiquement déformable entoure le support d'objectif, qui est rigide et lié rigidement (par exemple via le manche) à la partie principale en matière rigide, les déformations de la partie d'extrémité sont limitées par le support d'objectif.

Ainsi, malgré son caractère élastiquement déformable, la partie d'extrémité ne peut se déformer au point que ses déformations gênent la pénétration de l'appareil.

On bénéficie ainsi tout à la fois d'une résistance à la déformation qui permet l'introduction de l'extrémité du tube spéculum et d'une capacité de déformation suffisante pour que la partie d'extrémité se conforme au mieux à l'anatomie du vagin.

Cette aptitude de la partie d'extrémité du tube speculum à résister à une déformation excessive tout en étant capable de se déformer suffisamment pour se conformer à l'anatomie du vagin permet d'amorcer au mieux la pénétration du tube speculum.

La tâche de l'opérateur est ainsi facilitée et les risques de blessures de l'animal sont diminués, y compris dans les configurations anatomiques délicates telles que la présence d'irrégularités ou de renflements.

Selon des caractéristiques avantageuses, ladite partie d'extrémité présente une surface externe dont le diamètre augmente entre l'ouverture et la partie principale.

Le fait que le diamètre de la surface externe de la partie d'extrémité augmente depuis l'ouverture procure une progressivité qui facilite l'introduction du tube speculum.

L'appareil peut ainsi être utilisé avec une plus grande variété d'animaux qu'auparavant.

L'appareil selon l'invention peut en particulier être utilisé avec les génisses n'ayant encore jamais vêlé, ou les représentantes de la race buffaline, comme celles du genre *Bubalus bubalis* (Buffle d'Asie) et *Syncerus caffer* (Buffle d'Afrique), chez lesquelles les difficultés d'amorçage de la pénétration ne pouvaient être résolues jusqu'ici qu'en diminuant le diamètre du tube speculum.

Selon des caractéristiques avantageuses, ladite partie d'extrémité présente une surface interne dont le diamètre augmente entre l'ouverture et la partie principale et qui est contigüe audit support d'objectif.

La partie d'extrémité présente ainsi à partir de l'ouverture une paroi qui diverge, et qui est longée intérieurement par le support d'objectif, qui est donc décentré et incliné vers l'ouverture, laquelle est coaxiale au reste du tube speculum.

Selon des caractéristiques avantageuses, le contour de l'ouverture présente une zone la moins distale et une zone axialement en débord par rapport à la zone la moins distale, avec la zone la moins distale qui est à une position angulaire centrée sur le support d'objectif.

On bénéficie ainsi tout à la fois d'une progressivité supplémentaire à l'insertion (la zone axialement en débord pénètre avant la zone la moins distale) et d'un champ de vision par l'objectif qui n'est pas gêné par la paroi de la partie d'extrémité.

Selon d'autres caractéristiques avantageuses :
- ledit appareil comporte en outre un support de guidage dudit outil, ladite partie d'extrémité étant également disposée autour dudit support de guidage ;
- ledit support d'objectif et ledit support de guidage sont d'une seule pièce ;
- le support de guidage comporte une paroi en entonnoir coaxiale au tube speculum et des parois s'étendant chacune à partir de la paroi en entonnoir jusqu'à une extrémité adjacente à la surface interne du tube spéculum ; et le support d'objectif comporte un fût saillant vers l'ouverture depuis une zone périphérique du support de guidage jusqu'à une extrémité distale où il présente une ouverture, ledit objectif étant reçu dans le fût et situé au bord de l'ouverture du fût ;
- le tube speculum comporte une zone annulaire où la partie principale et la partie d'extrémité se recouvrent ;
- dans ladite zone annulaire, ladite partie d'extrémité présente des tétons saillant de sa surface externe tandis que la partie principale présente des orifices débouchant sur sa surface interne, chaque dit téton étant engagé dans un orifice respectif ; et/ou
- dans ladite zone annulaire, le jeu entre la partie d'extrémité et une paroi liée rigidement audit support d'objectif est, au droit d'au moins un dit téton, plus petit que l'épaisseur de ce téton.

### BREVE DESCRIPTION DES DESSINS

L'exposé de l'invention sera maintenant poursuivi par la description détaillée d'un exemple de réalisation, donnée ci-après à titre illustratif et non limitatif, en référence aux dessins annexés. Sur ceux-ci :
- la figure 1 est une vue de côté d'un ensemble de travail comportant un appareil d'aide à la pénétration vaginale conforme à l'invention et un outil de travail partiellement reçu dans l'appareil, l'ensemble étant dans une configuration initiale où l'outil de travail est dans une position reculée sur le manche de l'appareil, dans laquelle l'extrémité de l'outil de travail est située à l'intérieur du tube spéculum de l'appareil ;
- la figure 2 est une vue schématique en coupe longitudinale d'une paillette pour la conservation d'une dose prédéterminée de substance à base liquide, notamment de la semence animale pure ou diluée, convenant pour être utilisée avec l'outil de travail ;
- la figure 3 est une vue en coupe longitudinale de la portion d'extrémité de l'ensemble que l'on voit à droite sur la figure 1, montrant l'outil de travail avec la paillette de conservation de la figure 2 reçue à l'intérieur, le système de vision vidéo et un bloc support configuré pour guider à coulissement l'outil de travail et maintenir l'objectif du système de vision vidéo dans une position prédéterminée ;
- la figure 4 est une vue similaire à la figure 1 mais montrant l'ensemble dans une configuration intermédiaire où l'outil de travail est dans une position avancée sur le manche de l'appareil, dans laquelle l'extrémité de l'outil de travail saille en avant du tube speculum ;
- la figure 5 est une vue similaire à la figure 4 mais montrant l'ensemble dans une configuration finale où la semence a été éjectée hors de la paillette et de l'outil de travail ;
- la figure 6 est une vue en perspective du bloc support en cours de coopération avec l'outil de travail, le tube speculum ayant été enlevé ; et
- les figures 7 à 9 sont des vues de la partie d'extrémité du tube speculum montrée seule et respectivement en perspective, puis en plan de dessus, puis en plan de dessous.

### DESCRIPTION DETAILLEE D'UN EXEMPLE DE REALISATION

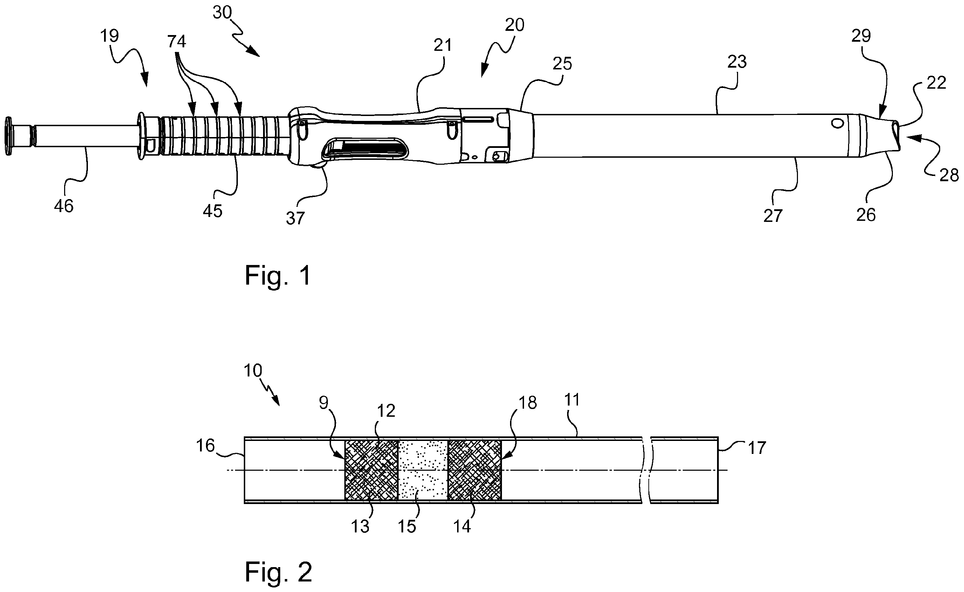

Les figures 1 à 3 illustrent un ensemble de travail 30 comportant un appareil 20 d'aide à la pénétration vaginale, un outil de travail 19 reçu à coulissement dans l'appareil 20 et une paillette 10 de conservation d'une dose prédéterminée de substance à base liquide, ici de la semence animale pure ou diluée (figure 2) que l'on utilise avec l'outil de travail 19.

L'outil de travail 19 est ici utilisé pour l'insémination artificielle d'animaux d'élevage avec la semence contenue dans la paillette 10.

Comme expliqué ci-après, l'appareil 20 permet de faciliter l'introduction de l'outil de travail 19 dans le vagin de l'animal et le guidage de l'outil de travail 19 jusqu'au col de l'utérus, où la semence doit être injectée.

L'appareil 20 et l'outil de travail 19 sont ici adaptés aux bovins.

L'appareil 20 comporte un manche 21 et un tube spéculum 23 s'étendant axialement depuis le manche 21 jusqu'à une extrémité distale 22 où il présente une ouverture 28.

Le manche 21 est formé par un corps tubulaire ouvert à ses deux extrémités.

Le tube spéculum 23 est ici coaxial au manche 21.

L'appareil 20 présente ainsi une forme généralement cylindrique allongée.

Le tube spéculum 23 est fixé au manche 21 par l'intermédiaire d'une bague de fixation 25.

La bague 25 est fixée au voisinage de l'extrémité proximale du tube speculum 23 (l'extrémité tournée vers le manche 21), ici par collage.

La bague de fixation 25 est attachée à l'extrémité distale du manche 21 (l'extrémité tournée vers le tube speculum 23), ici par encliquetage.

La bague 25 et le manche 21 sont configurés pour que la bague 25 puisse être désencliquetée du manche 21, le tube speculum 23 étant ainsi amovible.

Le tube speculum 23 comporte une partie d'extrémité 26, délimitant l'ouverture 28, et une partie principale 27 s'étendant entre la partie d'extrémité 26 et le manche 21.

Autrement dit la partie d'extrémité 26 s'étend entre la partie principale 27 et l'extrémité distale 22 du tube speculum 23 qui est aussi l'extrémité distale de la partie d'extrémité 26.

L'ouverture 28 est délimitée par un contour 31 correspondant à la tranche de la partie d'extrémité 26 située à l'extrémité distale 22.

La partie d'extrémité 26 est en matière élastiquement déformable, tandis que la partie principale 27 est en matière rigide.

La matière de la partie d'extrémité 26 comporte ici un matériau thermoplastique élastomère (ou TPE) qui a une dureté d'environ 40 Shore D. D'une manière générale, la matière de la partie d'extrémité 26 a une dureté comprise entre 35 et 45 Shore D.

La matière de la partie d'extrémité 26 a une limite d'allongement élastique qui est ici d'environ 20%. D'une manière générale, cette limite d'allongement élastique peut être égale ou supérieure à environ 20%.

La partie d'extrémité 26 est ici réalisée d'une seule pièce.

La matière de la partie principale 27 comporte ici du PMMA, qui est un matériau thermoplastique rigide transparent.

On observera que la partie d'extrémité 26 présente une surface externe 29 dont le diamètre augmente entre l'ouverture 28 et la partie principale 27.

Plus précisément, ce diamètre augmente jusqu'à atteindre le diamètre externe de la partie principale 27 qui, lui, reste constant le long de la partie principale 27.

En outre, la surface externe 29 augmente de diamètre de telle manière qu'elle présente un profil incurvé concave dont la concavité est tournée vers l'extérieur du tube 23.

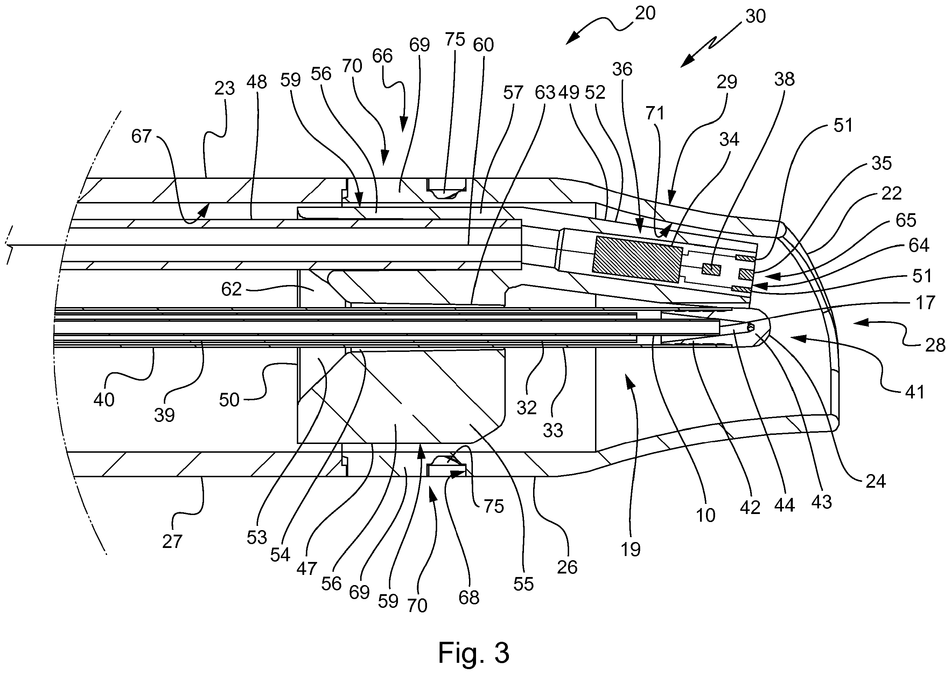

L'appareil 20 comporte en outre un système de vision vidéo comportant un objectif 35 (figure 3) disposé dans le tube speculum 23 et un dispositif de transmission 36 relié à l'objectif 35 et reliable à un écran déporté (non illustré) de visualisation de l'image obtenue par l'objectif 35.

Le dispositif de transmission 36 comporte un circuit électronique 34, un capteur photosensible 38 connecté au circuit électronique 34, une prise 37 et un câble 60 reliant le circuit électronique 34 à la prise 37.

Le capteur photosensible 38 est disposé derrière l'objectif 35 pour recevoir l'image obtenue par ce dernier et est configuré pour convertir cette image en un signal analogique ensuite transmis au circuit électronique 34. Le capteur photosensible 38 est ici du type CCD.

Le câble 60 est configuré pour acheminer le signal analogique depuis le circuit électronique 34 jusqu'à la prise 37 qui est logée dans la paroi du manche 21, à l'arrière de ce dernier (c'est-à-dire au voisinage de l'extrémité du manche opposée au tube speculum).

Le système de vision vidéo comporte en outre un ou plusieurs organes d'éclairage 51, ici des organes d'éclairage à LED, connectés au circuit électronique 34. Les organes d'éclairage 51 sont juxtaposées à l'objectif 35 et disposées de part et d'autre de ce dernier.

Le câble 60 et la prise 37 sont ici chacun configurés pour pouvoir à la fois acheminer le signal analogique et l'énergie nécessaire au fonctionnement du circuit électronique 34, du capteur 38 et des organes d'éclairage 51.

La prise 37 est ici un connecteur du type micro-USB.

Pour simplifier les dessins, le circuit électronique 34, le capteur 38, le câble 60, les organes d'éclairage 51 et l'objectif 35 sont représentés de manière très schématique sur la figure 3.

L'écran déporté fait ici partie d'un smartphone (non illustré) configuré pour être connecté au dispositif de transmission 36 à l'aide d'un câble (non illustré) approprié relié à la fois au smartphone et à la prise 37.

L'appareil 20 comporte en outre un bloc support 47 et un barreau de montage 48 chacun reçus dans le tube speculum 23.

Le barreau 48 est rigidement fixé par chacune de ses extrémités au manche 21 et au bloc support 47 respectivement, ce dernier occupant ainsi une position prédéterminée fixe dans le tube speculum 23.

Le barreau 48 est ici tubulaire et accueille le câble 60. En sortie du barreau 48, du côté du manche 21, le câble 60 circule à travers la paroi du manche 21 jusqu'à la prise 37.

Le bloc support 47 comporte un support d'objectif 49 et un support de guidage 50 de l'outil de travail 19, qui sont ici d'une seule pièce.

Le support d'objectif 49 maintient l'objectif 35 dans une position prédéterminée par rapport au tube speculum 23 dans laquelle l'objectif 35 est au voisinage de l'ouverture 28 et en regard de l'espace situé au-delà de cette ouverture 28, c'est-à-dire l'espace situé dans le prolongement du tube 23.

Le support d'objectif 49 maintient en outre les organes d'éclairage 51, qui sont configurés pour éclairer l'espace situé au-delà de l'ouverture 28.

On observera que la partie d'extrémité 26 du tube spéculum 23 est disposée autour du support d'objectif 49.

Le support de guidage 50 est configuré pour guider le coulissement de la partie de l'outil de travail 19 qui est reçue dans le tube spéculum 23. Le support de guidage 50 est en outre configuré pour que l'outil de travail 19 se déplace à coulissement selon une direction axiale centrée par rapport à la surface externe du tube speculum 23.

On observera que la partie d'extrémité 26 du tube speculum 23 est également disposée autour du support de guidage 50.

D'une manière générale, la partie d'extrémité 26 du tube speculum 23 est disposée autour du bloc support 47.

L'outil de travail 19 comporte ici une rallonge 45, un piston 46 monté à coulissement dans la rallonge 45, un pistolet d'insémination 32 réutilisable et une gaine sanitaire 33 à usage unique (partiellement illustrées sur la figure 3) que l'on utilise avec la paillette 10 (figures 2 et 3).

La rallonge 45 est configurée pour être montée à coulissement dans le manche 21 et est pourvue sur sa surface externe d'une pluralité de gorges annulaires 74 régulièrement espacées d'un pas prédéterminé. Les gorges annulaires 74 sont configurées pour coopérer avec un bourrelet (non illustré) ménagé sur la surface interne du manche 21 de sorte à former des crans de positionnement de l'outil de travail 19 par rapport à l'appareil 20. Le pas prédéterminé est ici d'environ 1 cm.

L'outil de travail 19 est reçu dans l'appareil 20 avec la rallonge 45 qui est montée à coulissement dans le manche 21, tandis que la partie de l'outil de travail 19 qui est reçue dans le tube speculum 23 est coulissante au travers du support de guidage 50.

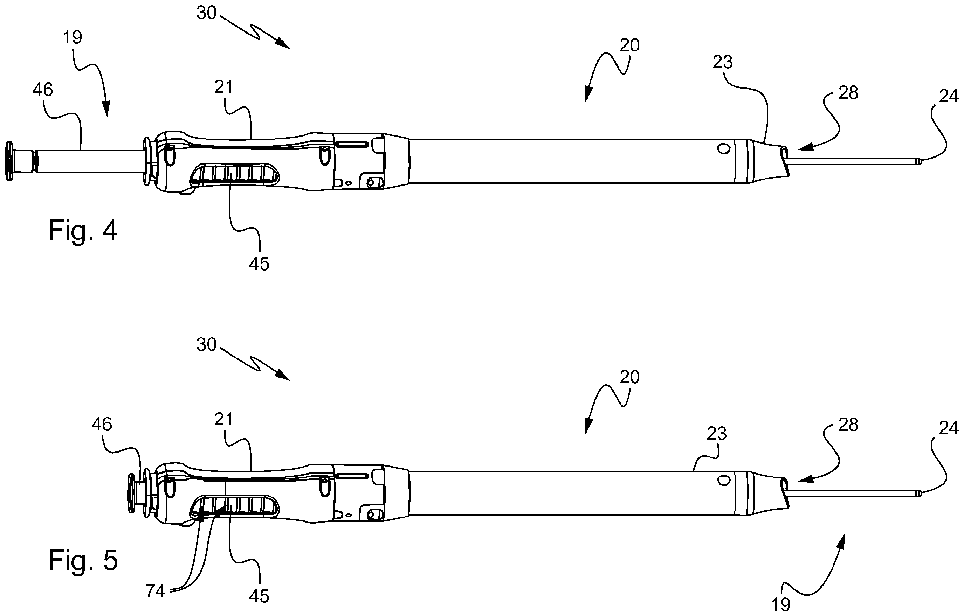

L'outil de travail 19 est ainsi monté mobile en translation par rapport à l'appareil 20 entre une position reculée (figures 1 et 3) par rapport à l'appareil 20, dans laquelle une extrémité distale 24 de l'outil de travail 19 est située à l'intérieur du tube speculum 23 (c'est-à-dire dans l'espace interne au tube 23 délimité par sa paroi), et une position avancée par rapport à l'appareil 20 (figures 4 et 5), dans laquelle l'extrémité distale 24 de l'outil 19 saille en avant du tube speculum 23.

Dans la position reculée, l'extrémité distale 24 de l'outil 19 se trouve donc du côté du tube 23 par rapport à l'ouverture 28, c'est-à-dire en arrière de l'ouverture 28.

Dans la position avancée, l'extrémité distale 24 de l'outil 19 se trouve donc du côté opposé au tube 23 par rapport à l'ouverture 28, c'est-à-dire en avant de l'ouverture 28.

Sur la figure 3, on peut observer la paillette 10 reçue dans l'outil de travail 19.

On va décrire plus en détails cette paillette 10 en référence à la figure 2.

La paillette 10 illustrée sur la figure 2 comporte un tube 11 et un bouchon 12.

Le tube 11 est classiquement en matière plastique extrudée, avec un diamètre interne qui est par exemple de 1,6 ou de 2,5 mm et une longueur de l'ordre de 133 mm.

Le bouchon 12 est habituellement du type tripartite décrit à l'origine dans le brevet français 995.878, correspondant au brevet britannique 669,265, c'est-à-dire formé par deux tampons 13 et 14 d'une substance fibreuse enserrant une poudre 15 susceptible de se transformer au contact d'un liquide en une pâte ou gel imperméable adhérant à la paroi du tube pour que le bouchon soit étanche aux liquides.

A l'état initial, illustré sur la figure 2, le bouchon 12 est disposé au voisinage de l'extrémité 16 du tube 11 et il est prévu qu'à l'état rempli, la dose de substance liquide qui doit être conservée dans la paillette 10 soit disposée entre le bouchon 12 et l'extrémité 17 du tube 11 la plus éloignée du bouchon 12.

Pour remplir la paillette 10, l'extrémité 16 est mise en communication avec une source de vide tandis que l'extrémité 17 est mise en communication avec un récipient contenant la substance à introduire dans la paillette.

L'air initialement contenu entre le bouchon 12 et l'extrémité 17 est aspiré au travers du bouchon tandis que la substance progresse dans le tube 11 jusqu'à ce qu'elle rencontre le bouchon 12, par l'extrémité 18 de celui-ci tournée vers l'extrémité 17 du tube 11, c'est-à-dire l'extrémité du bouchon 12 que l'on voit à droite sur la figure 2.

Le cas échéant, la paillette est soudée au voisinage de l'une ou de ses deux extrémités 16 et 17 et est stockée au froid.

Pour vider la paillette 10, le cas échéant après découpage des portions d'extrémité soudées et décongélation, on fait pénétrer dans le tube 11 une tige qui vient porter contre l'extrémité 9 du bouchon 12 (extrémité située du côté opposé à l'extrémité 18). Avec cette tige, on fait coulisser le bouchon 12 à la façon d'un piston vers l'extrémité 17 ou l'extrémité correspondante après découpe de la portion soudée, ce qui provoque l'expulsion de la dose de substance qui avait été introduite dans la paillette.

On va maintenant décrire plus en détails l'outil de travail 19 et sa coopération avec la paillette 10.

Le pistolet d'insémination 32 (figure 3) de l'outil de travail 19 comporte un corps tubulaire rigide 39, pour recevoir la paillette 10 remplie de semence, et comporte la tige d'entraînement du bouchon 12, montée coulissante dans le corps tubulaire rigide 39.

La rallonge 45 de l'outil de travail 19 est assujettie au corps tubulaire rigide 39 du pistolet 32, tandis que le piston 46 est assujetti à la tige du pistolet 32.

Le coulissement du piston 46 par rapport à la rallonge 45 entraîne ainsi le coulissement de la tige par rapport au corps tubulaire rigide 39 du pistolet 32.

Avant introduction de la paillette 10 dans le corps tubulaire rigide 39, l'inséminateur sort le piston 46 au maximum hors de la rallonge 45, afin que la tige soit sortie ou reculée au maximum hors du corps 39 du côté de l'extrémité proximale, c'est-à-dire du côté de l'extrémité qui est manipulée par l'inséminateur pendant l'opération, puis la paillette 10 est introduite dans le corps tubulaire rigide 39 à son extrémité distale (extrémité située du côté opposé à l'extrémité proximale), la paillette 10 étant introduite avec l'extrémité 16 du tube 11 (extrémité la plus proche du bouchon 12), la première. La paillette 10 s'enfonce dans le corps tubulaire rigide 39 du pistolet 32 jusqu'à ce que l'extrémité 16 du tube 11 rencontre un épaulement formant butée d'enfoncement.

La paillette 10 est alors en place dans le corps tubulaire rigide 39 du pistolet 32. L'extrémité 17 du tube 11 ainsi qu'une certaine longueur du tube 11 à partir de cette extrémité restent à l'extérieur du corps tubulaire rigide 39, c'est-à-dire qu'une certaine partie de la paillette 10 saille au-delà de l'extrémité distale du corps tubulaire rigide 39 du pistolet 32.

La gaine sanitaire 33 (figure 3) comporte un tube 40 dont le diamètre interne est tel que le corps tubulaire rigide 39 du pistolet 32 peut y être introduit. À une extrémité (extrémité proximale) le tube 40 de la gaine sanitaire 33 est ouvert et à l'autre extrémité (extrémité distale) la gaine sanitaire 33 comporte un embout 41 assujetti au tube 40.

Cet embout 41 comporte un pied 42 introduit dans une portion d'extrémité du tube 40 et une tête 43 disposée dans le prolongement du tube 40.

L'embout 41 comporte un conduit 44 débouchant dans le tube 40 à l'extrémité proximale du pied 42 (extrémité du pied située du côté opposé à la tête) et hors de la gaine 33 à la surface externe de la tête 43.

Ce conduit 44 interne à l'embout 41 comporte une portion orientée suivant la direction axiale, se rétrécissant depuis l'extrémité proximale du pied 42, configurée pour que la portion du tube 11 de la paillette 10 située au voisinage de l'extrémité 17 (extrémité la plus éloignée du bouchon 12) puisse s'introduire dans la portion du conduit 44 se rétrécissant et progresser jusqu'à une position de butée où la portion du tube 11 située au voisinage de l'extrémité 17 est enserrée par la paroi de cette portion du conduit 44.

Cet enserrement procure tout à la fois la butée d'enfoncement de la paillette 10 dans la gaine 33 et l'étanchéité aux liquides entre la paillette 10 et la gaine 33.

Le corps tubulaire rigide 39 du pistolet 32, dans lequel a été préalablement mise en place la paillette 10, est introduit dans la gaine 33 par son extrémité ouverte avec la paillette 10 la première et la paillette s'introduit dans le conduit 44 de l'embout 41. L'introduction dans la gaine 33 se termine lorsque la paillette 10 vient en butée contre la paroi du conduit 44 se rétrécissant.

La gaine 33 est alors fixée au corps tubulaire rigide 39 du pistolet 32, en général au voisinage de l'extrémité proximale de la gaine 33 (extrémité ouverte du tube 40) par exemple avec une bague appropriée.

Le tube 11 de la paillette 10 est ainsi immobilisé par rapport à l'ensemble formé par le corps tubulaire 39 du pistolet 32 et par la gaine sanitaire 33 fixée à celui-ci, puisque l'extrémité 16 du tube 11 est en butée contre l'épaulement du corps tubulaire 39 du pistolet 32 et l'extrémité 17 est en butée contre la paroi de la portion du conduit 44 se rétrécissant.

On peut alors utiliser le piston 46 pour entraîner la tige et faire coulisser le bouchon 12 de la paillette 10 afin d'éjecter la semence hors du tube 11 et hors du tube 40 de la gaine 33 par le conduit 44 qui débouche hors de la gaine 33 à la surface externe de la tête 43.

Pour pratiquer l'insémination, l'appareil 20 doit bien entendu avoir été préalablement introduit dans l'animal, puis l'outil de travail 19 monté sur l'appareil 20.

L'appareil 20 est introduit dans l'animal par l'inséminateur qui peut surveiller la progression du tube speculum 23 sur l'écran déporté.

On notera que le fait que le diamètre de la surface externe 29 de la partie d'extrémité 26 augmente depuis l'ouverture 28 procure une progressivité qui facilite l'introduction du tube speculum 23.

Une fois que l'appareil 20 est en place, c'est-à-dire lorsque l'ouverture 28 du tube 23 est en regard du col de l'utérus, l'outil de travail 19 est introduit au travers du manche 21, l'extrémité distale 24 la première, et avancé jusqu'à ce que la rallonge 45 soit enfoncée dans le manche 21 jusqu'au premier cran.

Au cours de l'introduction de l'outil 19, l'extrémité distale 24 de la gaine 33 traverse entièrement le manche 21, puis s'introduit dans le tube speculum 23 et passe au travers du support de guidage 50.

L'ensemble 30 parvient alors à la configuration illustrée sur la figure 1, qui est une configuration initiale où l'outil de travail 19 est dans la position reculée par rapport à l'appareil 20 et le piston 46 est sorti au maximum hors de la rallonge 45 (la semence n'a pas encore été éjectée).

La rallonge 45 est alors poussée en avant, cran par cran, pour la faire coulisser dans le manche 21 jusqu'à ce que l'outil de travail 19 arrive en position avancée.

Au cours de ce mouvement, l'extrémité distale 24 de l'outil 19 passe au travers de l'ouverture 28 du tube 23 et progresse dans le vagin de l'animal jusqu'à s'introduire dans le col de l'utérus.

L'inséminateur peut surveiller la progression de l'extrémité distale 24 sur l'écran déporté et ainsi veiller à ne pas blesser l'animal.

Le coulissement cran par cran de la rallonge 45 par rapport au manche 21 permet également de contrôler la progression de l'extrémité distale 24.

On notera que l'objectif 35 est maintenu par le support 49 dans une position prédéterminée telle que l'extrémité distale 24 entre dans le champ de vision de l'objectif 35 (et devient donc visible sur l'écran déporté) avant d'avoir franchi l'ouverture 28. Ainsi, tant que l'inséminateur ne distingue pas l'extrémité distale 24 sur l'écran, il est assuré qu'aucune partie de l'outil 19 ne dépasse de l'ouverture 28 et saille dans le vagin. En effet, une telle partie de l'outil 19 qui dépasserait de l'ouverture 28 risquerait de blesser l'animal lors d'éventuels mouvements incontrôlés de l'appareil 20.

On notera encore que puisque l'outil de travail 19 est centré par rapport à la surface externe du tube speculum 23, il est également centré par rapport au vagin. Ceci facilite l'introduction de l'outil 19 dans le col de l'utérus qui est lui aussi centré par rapport au vagin.

L'ensemble 30 parvient alors à la configuration illustrée sur la figure 4, qui est une configuration intermédiaire où l'outil de travail 19 est dans une position avancée et le piston 46 est encore sorti au maximum hors de la rallonge 45.

Il ne reste alors plus à l'inséminateur qu'à pousser le piston 46 pour éjecter la semence et terminer l'insémination.

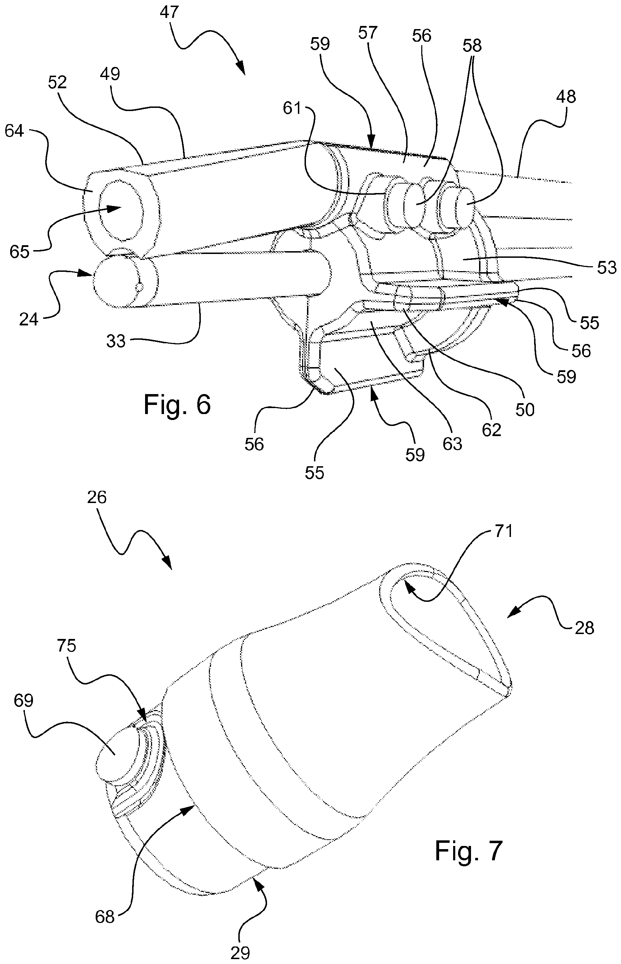

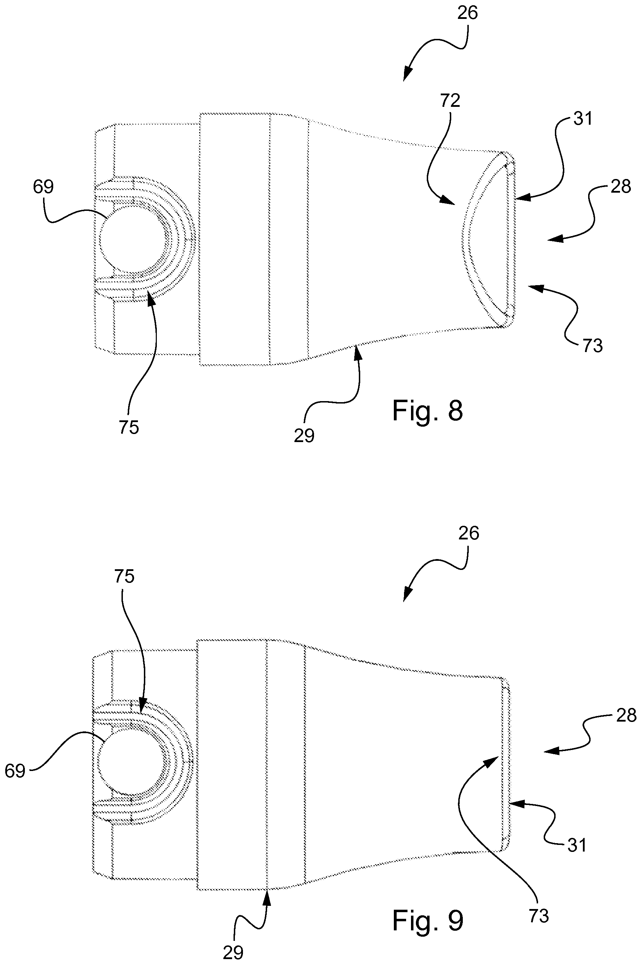

On va maintenant décrire plus en détails l'agencement de la partie avant de l'appareil 20, où se trouvent le bloc support 47 et la partie d'extrémité 26 du tube 23, à l'appui des figures 3 et 6 à 9.

Le support de guidage 50 comporte une paroi en entonnoir 53 coaxiale au tube speculum 23 et des parois 56 s'étendant chacune à partir de la paroi en entonnoir 53 jusqu'à une extrémité 59 adjacente à la surface interne du tube speculum 23.

La paroi en entonnoir 53 présente une partie conique 62, tournée vers le manche 21, et une partie droite 63 s'étendant depuis la partie conique 62 et tournée vers l'ouverture 28.

La paroi en entonnoir 53 délimite un passage 54 configuré pour recevoir à coulissement la partie de l'outil de travail 19 formée par la gaine 33 et le corps tubulaire rigide 39.

La partie conique 62 facilite l'introduction de la gaine 33 dans le passage 54 au moment de l'insertion de l'outil de travail 19 dans l'appareil 20.

Les extrémités 59 des parois 56 sont suffisamment proches de la surface interne du tube 23 pour que le support de guidage 50 conserve une position centrée vis-à-vis de la surface interne du tube spéculum 23, qui est une position à la fois centrée par rapport à la surface externe du tube spéculum 23 puisque ces surfaces interne et externe sont concentriques.

On notera que les parois 56 sont chacune liée rigidement au support d'objectif 49, ici puisque le support de guidage 50 et le support d'objectif 49 sont fait d'une seule pièce.

Les parois 56 forment ici trois ailettes 55 et un manchon 57. Deux des trois ailettes 55 sont situées à l'opposé l'une de l'autre par rapport à la paroi en entonnoir 53. Une autre des trois ailettes 55 et le manchon 57 sont situés à l'opposé l'un de l'autre par rapport à la paroi en entonnoir 53.

Les ailettes 55 et/ou le manchon 57 sont angulairement espacés les uns des autres d'environ un quart de tour.

Les ailettes 55 s'étendent chacune selon un plan général respectif parallèle au tube speculum 23.

Le manchon 57 est généralement orienté parallèlement au tube speculum 23 et reçoit à l'intérieur une portion d'extrémité du barreau de montage 48. Le barreau de montage 48 est ici fixé rigidement au bloc support 47 par des vis 58 engagées dans des cheminées 61 saillant latéralement de la paroi 56 du manchon 57.

On notera que le manchon 57 se trouve dans une zone périphérique du support de guidage 50 et est décentré par rapport au tube speculum 23.

Le support d'objectif 49 comporte en outre un fût 52 saillant vers l'ouverture 28 depuis une zone périphérique du support de guidage 50 jusqu'à une extrémité distale 64 où il présente une ouverture 65.

L'objectif 35 est reçu dans le fût 52 en étant situé au bord de son ouverture 65.

On observera qu'ici le circuit électronique 34, le capteur photosensible 38 et les organes d'éclairage 51 sont tous reçus dans le fût 52.

Le fût 52 s'étend plus précisément depuis le manchon 57 qu'il prolonge, l'espace interne au fût 52 communicant avec celui du manchon 57.

En sortie du barreau 48, du côté du bloc support 47, le câble 60 circule dans le fût 52 jusqu'au circuit électronique 34.

On notera que le fût 52 est décentré par rapport au tube speculum 23 et que le fût 52 s'étend selon une direction inclinée vers l'ouverture 28 du tube speculum 23 et vers l'intérieur du tube speculum 23.

Dans la partie avant de l'appareil 20, le tube speculum 23 comporte une zone annulaire 66 où la partie principale 27 et la partie d'extrémité 26 se recouvrent.

La partie principale 27 présente une surface interne 67 qui délimite son espace interne.

Dans la zone annulaire 66, la partie d'extrémité 26 est emmanchée dans la partie principale 27 avec la surface externe 29 de la partie d'extrémité 26 qui est au contact de la surface interne 67 de la partie principale 27.

Dans la zone annulaire 66, la surface externe 29 de la partie d'extrémité 26 est en retrait par rapport à la partie de la surface externe 29 qui est en dehors de la zone annulaire 66.

De ce fait, la partie d'extrémité 26 présente un épaulement 68 qui vient porter contre la tranche de la partie principale 27 située à son extrémité distale (extrémité tournée vers l'ouverture 28).

En outre, dans la zone annulaire 66, la surface interne 67 de la partie principale 27 est en renfoncement par rapport à la partie de la surface interne 67 qui est en dehors de la zone annulaire 66.

Du fait des renfoncements respectifs de la surface externe 29 de la partie d'extrémité 26 et de la surface interne 67 de la partie principale 27 dans la zone annulaire 66, les surfaces interne et externe du tube speculum 23 restent lisses à la jonction entre les parties principale 27 et d'extrémité 26.

On notera que dans la zone annulaire 66, la partie principale 27 et la partie d'extrémité 26 sont toutes les deux disposées autour du support de guidage 50.

Dans la zone annulaire 66, la partie d'extrémité 26 présente deux tétons 69 saillant de sa surface externe 29 tandis que la partie principale 27 présente deux orifices 70 débouchant sur sa surface interne 67, chaque téton 69 étant engagé dans un orifice 70 respectif.

Les tétons 69 et les orifices forment des organes de verrouillage qui s'opposent à la fois à un mouvement axial et un mouvement de rotation de la partie d'extrémité 26 par rapport à la partie principale 27.

Chaque orifice 70 débouche en outre sur la surface externe du tube speculum 23, avec la surface du téton 69 engagé dans cet orifice 70 qui est à fleur de la surface externe du tube 23.

Les deux tétons 69 sont situés diamétralement à l'opposé l'un de l'autre sur la partie d'extrémité 26 ; tandis que les deux orifices 70 sont situés diamétralement à l'opposé l'un de l'autre sur le tube speculum 23.

En outre, dans la zone annulaire 66, chacun des couples formé par un orifice 70 et un téton 69 engagé dans l'orifice 70, est situé angulairement au droit d'une paroi 56 respective du support de guidage 50 avec, au droit de chaque téton 69, le jeu entre la partie d'extrémité 26 et la paroi 56 respective qui est plus petit que l'épaisseur de ce téton 69.

Il est ainsi impossible à un téton 69 de se désengager de l'orifice 70 dans lequel il est reçu tant que le bloc support 47, et en particulier le support de de guidage 50, est en place dans le tube 23 au droit de la zone annulaire 66.

Pour démonter la partie d'extrémité 26, la bague de fixation 25 doit d'abord être désencliquetée pour pouvoir séparer le tube 23 du manche 21. Le tube 23 est ensuite écarté du manche 21 de sorte que le bloc support 47 ne se trouve plus au droit de la zone annulaire 66.

Les tétons 69 peuvent alors être pressés vers l'intérieur du tube 23 pour être désengagés des orifices 70.

Pour faciliter le désengagement et/ou l'engagement des tétons 69 dans les orifices 70, la partie d'extrémité 26 présente en outre des gorges 75 en forme de U ménagées dans sa surface externe 29 au pied de chaque téton 69 en entourant ce dernier.

La partie d'extrémité 26 présente en outre une surface interne 71 dont le diamètre augmente entre l'ouverture 28 et la partie principale 27, le diamètre de l'ouverture 28 étant plus petit que le diamètre interne de la partie principale 27, et l'ouverture 28 étant coaxiale au tube speculum 23.

Le diamètre de la surface interne 71 augmente jusqu'à avoir le même diamètre que la surface interne 67 de la partie principale 27 (hors zone annulaire 66).

La surface interne 71 est en outre contiguë au fût 52 du support d'objectif 49, qui est décentré et incliné comme déjà expliqué.

On observera que la paroi de la partie d'extrémité 26 est plus mince dans la partie où ses surfaces interne 71 et externe 29 augmentent de diamètre, qu'entre cette partie et l'épaulement 68. Entre cette partie et l'épaulement 68, la paroi présente la même épaisseur que la partie principale 27.

Le contour 31 de l'ouverture 28 présente une zone 72 la moins distale et une zone 73 axialement en débord par rapport à la zone 72 la moins distale, avec la zone 72 la moins distale qui est à une position angulaire centrée sur le support d'objectif 49.

Autrement dit, le contour 31 de l'ouverture 28 est axialement plus proche de l'ouverture 65 du fût 52 dans la zone 72 que dans la zone 73.

Le champ de vision par l'objectif 35 n'est ainsi pas gêné par la paroi de la partie d'extrémité 26.

La proximité de l'ouverture 65 du fût avec l'ouverture 28 du tube 23 permet également d'éviter qu'une portion de la partie d'extrémité 26 qui est axialement en avant de l'extrémité 64 du fût 52 (et donc n'entoure pas le fût 52) ne se rabatte devant l'objectif 35 et obstrue son champ de vision, en se déformant lors de l'introduction du tube 23 dans l'animal.

Dans des variantes non illustrées :
- l'outil de travail est dépourvu d'un pistolet d'insémination et comporte par exemple une pince de prélèvement, ou un dispositif d'écoute ;
- la gaine sanitaire est dépourvue d'un embout et présente à la place un bord retourné formant ourlet à son extrémité distale et un manchon coulissant disposé à l'intérieur de la gaine, le manchon étant configuré pour recevoir une extrémité de la paillette et assurer l'étanchéité entre la gaine et la paillette lorsque la paillette pousse le manchon en butée contre l'ourlet ;
- le système de vision vidéo ne comporte qu'un seul organe d'éclairage juxtaposé à l'objectif ;
- le capteur photosensible n'est pas du type CCD mais est par exemple du type CMOS ;
- le dispositif de transmission du système de vision vidéo est dépourvu d'un circuit électronique, d'un capteur photosensible et d'un câble mais comporte plutôt un câble à fibres optiques ;
- le dispositif de transmission est dépourvu d'une prise pour y raccorder un écran déporté et comporte à la place un dispositif de connexion sans fil, par exemple un émetteur/récepteur radio de type Wi-Fi ou Bluetooth ;
- le dispositif de transmission comporte un projecteur configuré pour projeter l'image sur un support formant l'écran déporté, le support étant par exemple un mur ou une toile ;
- l'écran déporté n'est pas celui d'un smartphone mais celui d'une tablette, d'un PC, de lunettes et/ou d'un projecteur ;
- la bague de fixation du tube speculum sur le manche est dépourvues d'organes d'encliquetage et comporte plutôt des organes de fixation du type baïonnette ;
- la matière du tube speculum est différente d'un matériau thermoplastique et est par exemple en verre ;
- le tube speculum n'est pas transparent mais translucide ou opaque ;
- la partie d'extrémité du tube speculum est assujettie de manière permanente à sa partie principale, par exemple en étant collée ;
- la partie d'extrémité du tube speculum n'est pas emmanchée dans sa partie principale mais y est aboutée ;
- la partie d'extrémité du tube speculum recouvre sa partie principale par l'extérieur ; et/ou
- la partie d'extrémité/la partie principale comportent respectivement plus ou moins que deux tétons/orifices, par exemple trois tétons/orifices chacun en regard d'une paroi respective du support de guidage.

De nombreuses autres variantes sont possibles en fonction des circonstances, et l'on rappelle à cet égard que l'invention ne se limite pas aux exemples décrits et représentés. L'invention n'est délimitée que par les revendications suivantes.

## Revendications

1. Appareil d'aide à la pénétration vaginale prévu pour recevoir un outil de travail, comportant :
- un manche (21) ;
- un tube speculum (23) s'étendant axialement depuis le manche (21) jusqu'à une extrémité distale (22) où il présente une ouverture (28) ;
- un système de vision vidéo comportant un objectif (35) disposé dans le tube spéculum (23) et un dispositif de transmission (36) relié à l'objectif (35) et reliable à un écran déporté de visualisation de l'image obtenue par ledit objectif (35) ; et
- un support (49) d'objectif pour maintenir l'objectif (35) dans une position prédéterminée par rapport au tube speculum (23) dans laquelle l'objectif (35) est au voisinage de ladite ouverture (28) du tube speculum (23) et en regard de l'espace situé au-delà de ladite ouverture (28) du tube speculum (23) ;
**caractérisé en ce que** ledit tube spéculum (23) comporte une partie d'extrémité (26) et une partie principale (27) s'étendant entre ladite partie d'extrémité (26) et ledit manche (21), ladite partie d'extrémité (26) étant disposée autour dudit support (49) d'objectif et délimitant ladite ouverture (28), ladite partie d'extrémité (26) étant en matière élastiquement déformable et ladite partie principale (27) étant en matière rigide.

2. Appareil selon la revendication 1, **caractérisé en ce que** ladite partie d'extrémité (26) présente une surface externe (29) dont le diamètre augmente entre l'ouverture (28) et la partie principale (27).

3. Appareil selon la revendication 2, **caractérisé en ce que** ladite partie d'extrémité (26) présente une surface interne (71) dont le diamètre augmente entre l'ouverture (28) et la partie principale (27) et qui est contiguë audit support (49) d'objectif.

4. Appareil selon l'une quelconque des revendications 1 à 3, **caractérisé en ce que** le contour (31) de l'ouverture (28) présente une zone (72) la moins distale et une zone (73) axialement en débord par rapport à la zone (72) la moins distale, avec la zone (72) la moins distale qui est à une position angulaire centrée sur le support (49) d'objectif.

5. Appareil selon l'une quelconque des revendications 1 à 4, **caractérisé en ce qu'**il comporte en outre un support de guidage (50) dudit outil (19), ladite partie d'extrémité (26) étant également disposée autour dudit support de guidage (50).

6. Appareil selon la revendication 5, **caractérisé en ce que** ledit support (49) d'objectif et ledit support de guidage (50) sont d'une seule pièce.

7. Appareil selon la revendication 6, **caractérisé en ce que** le support de guidage (50) comporte une paroi en entonnoir (53) coaxiale au tube speculum (23) et des parois (56) s'étendant chacune à partir de la paroi en entonnoir (53) jusqu'à une extrémité (59) adjacente à la surface interne du tube speculum (23) ; et le support (49) d'objectif comporte un fût (52) saillant vers l'ouverture (28) depuis une zone périphérique du support de guidage (50) jusqu'à une extrémité distale (64) où il présente une ouverture (65), ledit objectif (35) étant reçu dans le fût (52) et situé au bord de l'ouverture (65) du fût (52).

8. Appareil selon l'une quelconque des revendications 1 à 7, **caractérisé en ce que** le tube spéculum (23) comporte une zone annulaire (66) où la partie principale (27) et la partie d'extrémité (26) se recouvrent.

9. Appareil selon la revendication 8, **caractérisé en ce que** dans ladite zone annulaire (66), ladite partie d'extrémité (26) présente des tétons (69) saillant de sa surface externe (29) tandis que la partie principale (27) présente des orifices (70) débouchant sur sa surface interne (67), chaque dit téton étant engagé dans un orifice (70) respectif.

10. Appareil selon la revendication 9, **caractérisé en ce que** dans ladite zone annulaire (66), le jeu entre la partie d'extrémité (26) et une paroi (56) liée rigidement audit support (49) d'objectif est, au droit d'au moins un dit téton (69), plus petit que l'épaisseur de ce téton (69).

## Patentansprüche

1. Gerät zur Unterstützung der vaginalen Penetration und vorgesehen zum Aufnehmen eines Arbeitswerkzeug, Folgendes umfassend:
- einen Schaft (21);
- ein Spekulumrohr (23), das sich axial von dem Schaft (21) aus bis zu einem distalen Ende (22) erstreckt, wo es eine Öffnung (28) aufweist;
- ein Video-Bildverarbeitungssystem, enthaltend ein Objektiv (35), das in dem Spekulumrohr (23) angeordnet ist, und eine Übertragungsvorrichtung (36), die mit dem Objektiv (35) verbunden ist und mit einem abgesetzten Bildschirm zur Anzeige des von dem Objektiv (35) erhaltenen Bildes verbunden werden kann; und
- einen Objektivhalter (49) zum Halten des Objektivs (35) in einer vorher bestimmten Position in Bezug auf das Spekulumrohr (23), in der sich das Objektiv (35) in der Nähe der Öffnung (28) des Spekulumrohrs (23) befindet und dem Raum jenseits der Öffnung (28) des Spekulumrohrs (23) zugewandt ist;
**dadurch gekennzeichnet, dass** das Spekulumrohr (23) einen Endabschnitt (26) und einen Hauptabschnitt (27) aufweist, der sich zwischen dem Endabschnitt (26) und dem Schaft (21) erstreckt, wobei der Endabschnitt (26) um den Objektivhalter (49) herum angeordnet ist und die Öffnung (28) begrenzt, wobei der Endabschnitt (26) aus einem elastisch verformbaren Material besteht und der Hauptabschnitt (27) aus einem starren Material besteht.

2. Vorrichtung nach Anspruch 1, **dadurch gekennzeichnet, dass** der Endabschnitt (26) eine Außenfläche (29) aufweist, deren Durchmesser zwischen der Öffnung (28) und dem Hauptabschnitt (27) zunimmt.

3. Vorrichtung nach Anspruch 2, **dadurch gekennzeichnet, dass** der Endabschnitt (26) eine Innenfläche (71) aufweist, deren Durchmesser sich zwischen der Öffnung (28) und dem Hauptteil (27) vergrößert und die an den Objektivhalter (49) angrenzt.

4. Vorrichtung nach einem der Ansprüche 1 bis 3, **dadurch gekennzeichnet, dass** die Kontur (31) der Öffnung (28) einen am wenigsten distalen Bereich (72) und einen Bereich (73) aufweist, der in Bezug auf den am wenigsten distalen Bereich (72) axial hinausragt, wobei sich der am wenigsten distale Bereich (72) in einer Winkelposition befindet, die auf dem Objektivhalter (49) zentriert ist.

5. Vorrichtung nach einem der Ansprüche 1 bis 4, **dadurch gekennzeichnet, dass** sie außerdem einen Führungshalter (50) für das Werkzeug (19) aufweist, wobei der Endabschnitt (26) auch um den Führungshalter (50) herum angeordnet ist.

6. Vorrichtung nach Anspruch 5, **dadurch gekennzeichnet, dass** der Objektivhalter (49) und der Führungshalter (50) aus einem Stück bestehen.

7. Vorrichtung nach Anspruch 6, **dadurch gekennzeichnet, dass** der Führungshalter (50) eine trichterförmige Wand (53) koaxial zu dem Spekulumrohr (23) und Wände (56) aufweist, die sich jeweils von der trichterförmigen Wand (53) zu einem Ende (59) erstrecken, das an die Innenfläche des Spekulumrohrs (23) angrenzt; und der Objektivhalter (49) eine Hülse (52) aufweist, die in Richtung der Öffnung (28) von einem Umfangsbereich des Führungshalters (50) zu einem distalen Ende (64) ragt, wo er eine Öffnung (65) aufweist, wobei das Objektiv (35) in der Hülse (52) aufgenommen wird und an der Kante der Öffnung (65) der Hülse (52) angeordnet ist.

8. Vorrichtung nach einem der Ansprüche 1 bis 7, **dadurch gekennzeichnet, dass** das Spekulumrohr (23) einen ringförmigen Bereich (66) aufweist, in dem sich der Hauptteil (27) und der Endteil (26) überlappen.

9. Vorrichtung nach Anspruch 8, **dadurch gekennzeichnet, dass** in dem ringförmigen Bereich (66) der Endabschnitt (26) Zapfen (69) aufweist, die von seiner Außenfläche (29) vorstehen, während der Hauptabschnitt (27) Öffnungen (70) aufweist, die in seine Innenfläche (67) münden, wobei jeder der Zapfen in eine jeweilige Öffnung (70) eingreift.

10. Vorrichtung nach Anspruch 9, **dadurch gekennzeichnet, dass** in dem ringförmigen Bereich (66) der Abstand zwischen dem Endabschnitt (26) und einer Wand (56), die starr mit dem Objektivhalter (49) verbunden ist, an mindestens einem der Zapfen (69) kleiner ist als die Dicke dieses Zapfens (69).

## Claims

1. An apparatus for assisting in vaginal penetration provided to receive a work tool, comprising:
- a handling shaft (21);
- a speculum tube (23) extending axially from the handling shaft (21) to a distal end (22) at which it has an opening (28);
- a video viewing system comprising an objective (35) disposed within the speculum tube (23) and a transmission device (36) connected to the objective (35) and connectable to a remotely located screen for viewing the image obtained by said objective (35); and
- an objective support (49) to hold the objective (35) in a predetermined position relative to the speculum tube (23) in which the objective (35) is in the neighborhood of said opening (28) of the speculum tube (23) and faces the space located beyond said opening (28) of the speculum tube (23);
**characterized in that** said speculum tube (23) comprises an end part (26) and a main part (27) extending between said end part (26) and said handling shaft (21), said end part (26) being disposed around said objective support (49) and delimiting said opening (28), said end part (26) being of elastically deformable material and said main part (27) being of rigid material.

2. Apparatus according to claim 1, **characterized in that** said end part (26) has an outside surface (29) of which the diameter increases between the opening (28) and the main part (27).

3. Apparatus according to claim 2, **characterized in that** said end part (26) has an inside surface (71) of which the diameter increases between the opening (28) and the main part (27) and which is contiguous with said objective support (49).

4. Apparatus according to any one of claims 1 to 3, **characterized in that** the contour (31) of the opening (28) has a least distal zone (72) and a zone (73) which protrudes axially relative to the least distal zone (72), the least distal zone (72) being at an angular position centered on the objective support (49).

5. Apparatus according to any one of claims 1 to 4, **characterized in that** it further comprises a guiding support (50) for said tool (19), said end part (26) being also disposed around said guiding support (50).

6. Apparatus according to claim 5, **characterized in that** said objective support (49) and said guiding support (50) form a single part.

7. Apparatus according to claim 6, **characterized in that** the guiding support (50) comprises a funnel-shaped wall (53) coaxial with the speculum tube (23) and walls (56) each extending from the funnel-shaped wall (53) to an end (59) adjacent to the inside surface of the speculum tube (23); and the objective support (49) comprises a barrel (52) projecting towards the opening (28) from a peripheral zone of the guiding support (50) to a distal end (64) at which it has an opening (65), said objective (35) being received in the barrel (52) and located at the edge of the opening (65) of the barrel (52).

8. Apparatus according to any one of claims 1 to 7, **characterized in that** the speculum tube (23) comprises an annular zone (66) in which the main part (27) and the end part (26) overlap.

9. Apparatus according to claim 8, **characterized in that** in said annular zone (66), said end part (26) has nipples (69) projecting from its outside surface (29) while the main part (27) has orifices (70) opening onto its inside surface (67), each said nipple being engaged in a respective orifice (70).

10. Apparatus according to claim 9, **characterized in that** in said annular zone (66), the gap between the end part (26) and a wall (56) rigidly connected to said objective support (49) is, opposite at least one said nipple (69), smaller than the thickness of that nipple (69).
